(19) **Europäisches Patentamt**
**European Patent Office**
**Office européen des brevets**

(11) **EP 3 438 247 A1**

(12) **EUROPEAN PATENT APPLICATION**
published in accordance with Art. 153(4) EPC

(43) Date of publication:
**06.02.2019 Bulletin 2019/06**

(21) Application number: **17775129.4**

(22) Date of filing: **28.03.2017**

(51) Int Cl.:
***C12N 5/071*** (2010.01)     ***C12N 7/00*** (2006.01)

(86) International application number:
**PCT/JP2017/012726**

(87) International publication number:
**WO 2017/170592 (05.10.2017 Gazette 2017/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.03.2016 JP 2016065251**

(71) Applicant: **The Research Foundation for Microbial Diseases of Osaka University**
**Suita-shi, Osaka 565-0871 (JP)**

(72) Inventors:
• **UOTANI, Tae**
**Kanonji-shi**
**Kagawa 768-0065 (JP)**
• **KUWABARA, Soichiro**
**Kanonji-shi**
**Kagawa 768-0065 (JP)**

(74) Representative: **De Clercq & Partners**
**Edgard Gevaertdreef 10a**
**9830 Sint-Martens-Latem (BE)**

(54) **METHOD FOR CULTURING MDCK CELLS**

(57)     The present invention relates to a cloned MDCK cell showing an expansion factor of 4.5 or more when cultured using a microcarrier and a method of culturing the MDCK cell, a method of growing a virus using the method of culturing the MDCK cell, and a cloned MDCK cell showing an expansion factor of 4.5 or more when cultured using a microcarrier.

FIG. 2

**CULTURE TIME** (hr)

⎯△⎯ Pre Cloning Cell
⎯■⎯ **Cell Line A**
⎯●⎯ **Cell Line B**

Solid Line: Attached Viable Cell Density
Dashed Line: Floating Whole Cell Density

EP 3 438 247 A1

## Description

Technical Field

The present invention relates to a method of culturing an MDCK cell using a microcarrier and an MDCK cell suited for microcarrier culture. The present invention also relates to a method of growing a virus involving culturing an MDCK cell using a microcarrier.

The present application claims priority from Japanese Patent Application No. 2016-65251, which is incorporated herein by reference.

Background Art

In recent years, there has been a demand for efficient mass culture of cells, a tissue, microorganisms, or the like in various fields, such as pharmaceutical production and regenerative medicine. In particular, in order to put a biopharmaceutical or regenerative medicine into practical use, it is important to establish a mass culture technology for cells . Mass culture of cells is expected to significantly promote the putting of the biopharmaceutical or the like into practical use by enabling a transfer of a monoclonal antibody, a recombinant protein, a membrane protein, a virus, or the like to a production scale.

As a mass culture technology for cells having adhesion properties, there is known a microcarrier culture method. The microcarrier culture method is a method involving introducing cells, a culture medium, and a microcarrier, which serves as an adhesion scaffold for the cells, into a culture vessel, and intermittently stirring the culture medium to bring floating cells and the microcarrier into contact with each other, to thereby cause the cells to adhere to a surface of the microcarrier. This method allows the cells to grow on the surface of the microcarrier. The microcarrier can provide an extremely large surface area for the cells to adhere and grow relative to a volume ratio, and hence is suited for mass culture of cells.

As one of the fields in which the mass culture technology for cells is utilized, there is given vaccine manufacture. In the vaccine manufacture, a vaccine is manufactured by selecting host cells showing sensitivity to a virus of interest, then culturing the cells, and infecting the resultant large amount of cells with the virus to grow the virus . In order to put the vaccine into practical use, a large amount of the virus is needed, and hence the cells to be infected with the virus need to be prepared in a large amount.

Influenza is an infectious disease that causes a global outbreak every year, and may even cause a pandemic. Therefore, there is a need to secure a large amount of an influenza vaccine. For manufacture of the influenza vaccine, a method involving growing an influenza virus through utilization of an embryonated chicken egg has heretofore been used. The manufacturing method utilizing an embryonated chicken egg involves difficulties in terms of raw material procurement and quality control, and hence a manufacturing method involving growing an influenza virus in cultured cells is beginning to be put into practical use. However, it is considered a problem in that the cultured cells provide poor growth potential of the virus, and hence restrict the vaccine from being supplied rapidly and in a sufficient amount. In view of this, there is an urgent need to construct a system capable of producing a vaccine in a large amount and rapidly.

The manufacture of the influenza vaccine includes a stage of isolating or creating a seed virus of the influenza virus, a stage of preparing a required amount of embryonated chicken eggs or cultured cells for growing the seed virus, and a stage of growing the obtained seed virus using the embryonated chicken eggs, the cultured cells, or the like. The cultured cells may be used in each of the stages. As traditional cultured cells to be used for the manufacture of the influenza vaccine, there are given, for example, Madin-Darby Canine Kidney-derived cells (hereinafter referred to as MDCK cells) and African green monkey kidney-derived cells (hereinafter referred to as Vero cells). The Vero cells were isolated and established from kidney epithelial cells of an African green monkey (*Cercopithecus aethiops)* in 1962, and have been used for manufacture of vaccines for humans against infectious diseases, such as poliovirus and Japanese encephalitis virus, for 20 years or more. The Vero cells have sensitivity to a wide range of viruses, and are used for growth of the influenza virus as well. The MDCK cells are cells established from the kidney of a normal male Cocker Spaniel in 1958, and were revealed to have sensitivity to the influenza virus by Gaush et al. in 1968. Addition of trypsin, glucose, vitamin, and the like to a culture medium of the MDCK cells enhances the sensitivity to the influenza virus, and this has been utilized for isolation of various influenza viruses.

Attempts have been made to develop MDCK cells to be used in the stage of growing the influenza virus . For example, in Patent Literature 1, there is a disclosure that an MDCK cell line (ATCC CCL-34) was adapted to a serum-free medium to establish an MDCK-33016 line capable of suspension (floating) culture. In addition, in Patent Literature 2 and Patent Literature 3, there is a disclosure that a PTA-7909 line and a PTA-7910 line, each capable of replicating a cold-adapted influenza virus, were cloned from the MDCK cell line (ATCC CCL-34) . Meanwhile, MDCK cells infected with a virus produce an interferon that suppresses virus growth, and hence also have a property of making it hard for the virus to grow as compared to the Vero cells, which do not produce the interferon.

The microcarrier has been utilized for mass culture of cells, but it can hardly be said that a mass culture method for MDCK cells using a microcarrier has been established. In order for the MDCK cells to show high growth efficiency on the microcarrier, a strong adhesion force between the cells and the microcarrier is required. However, there is a problem in that repetition of cell passages for scale-up of a culture vessel weakens the adhesion force between the cells and the microcarrier. Further, culture of the MDCK cells using the microcarrier involves stirring, and hence it is considered that the cells are subjected to a physical stress through frequent contact of the cells with a stirring blade, a culture vessel wall, or the like. Consequently, there is a problem of a reduction in growth efficiency of the cells during the mass culture of the MDCK cells using the microcarrier.

In the mass culture of cells, a medium supplemented with serum of animal origin, such as bovine or horse serum, is generally used. The serum serves as a supply source of hormones, growth factors, and the like, and is also useful in relieving a physical stress due to a culture operation. When cells are cultured using the microcarrier, large amounts of hormones, growth factors, and the like are required to be supplied owing to growth of the cells in a large amount caused by increased efficiency of culture. However, the use of the serum raises concerns about safety and applicability to plant-scale production. Therefore, there is a demand for cells capable of growing in a serum-free medium in cell culture using the microcarrier.

Citation List

Patent Literature

[PTL 1] WO 1997/037000 A1
[PTL 2] WO 2008/105931 A2
[PTL 3] WO 2010/036760 A1

Summary of Invention

Technical Problem

An object of the present invention is to provide an MDCK cell suited for culture using a microcarrier and a method of culturing the MDCK cell.

Solution to Problem

The inventors of the present invention have made extensive investigations in order to achieve the above-mentioned object, and as a result, have found that an MDCK cell having not only a high cell growth ability in a serum-free medium, but also a strong adhesion force to a microcarrier, and a culture method using the cell are suited for culture using the microcarrier. The inventors have further found that the cell has a high expansion factor on the microcarrier in culture in the serum-free medium. Thus, the inventors have completed the present invention.

That is, the present invention includes the following.

1. A method of culturing a cloned MDCK cell showing an expansion factor of 4.5 or more when cultured using a microcarrier.

2. A method of culturing a cloned MDCK cell according to the above-mentioned item 1, wherein the cloned MDCK cell shows a cell floating ratio of 20% or less.

3. A method of culturing a cloned MDCK cell according to the above-mentioned item 1, wherein the expansion factor of the cloned MDCK cell includes an expansion factor in a case where the cloned MDCK cell is seeded at a cell seeding density of $2.0 \times 10^4$ cells/cm$^2$ or less and cultured.

4. A method of culturing a cloned MDCK cell according to the above-mentioned item 1 or 3, wherein the expansion factor of the cloned MDCK cell includes an expansion factor in a case where the cloned MDCK cell is cultured for 48 hours or more.

5. A method of culturing a cloned MDCK cell according to any one of the above-mentioned items 1 to 4, wherein the cloned MDCK cell includes a cell obtained by adapting an MDCK cell population to a serum-free medium, followed by cloning.

6. A method of culturing a cloned MDCK cell, including culturing an MDCK cell identified by accession number NITE BP-02014 using a microcarrier.

7. A method of growing a virus, including using the method of culturing a cloned MDCK cell of any one of the above-mentioned items 1 to 5.

8. A method of growing a virus according to the above-mentioned item 7, further including infecting an MDCK cell with a virus, and incubating the MDCK cell.

9. A method of growing a virus according to the above-mentioned item 7 or 8, wherein the virus includes an influenza virus.

10. A cloned MDCK cell showing an expansion factor of 4.5 or more when cultured using a microcarrier.

11. A cloned MDCK cell according to the above-mentioned item 10, wherein the expansion factor of the cloned MDCK cell includes an expansion factor in a case where the cloned MDCK cell is seeded at a cell seeding density of $2.0 \times 10^4$ cells/cm$^2$ or less and cultured.

12. A cloned MDCK cell according to the above-mentioned item 10 or 11, wherein the expansion factor of the cloned MDCK cell includes an expansion factor in a case where the cloned MDCK cell is cultured for 48 hours or more.

13. A cloned MDCK cell according to any one of the above-mentioned items 10 to 12, wherein the cloned MDCK cell includes a cell obtained by adapting an MDCK cell population to a serum-free medium, followed by cloning.

Advantageous Effects of Invention

According to the MDCK cell and the method of culturing the cell with a microcarrier of the present invention, MDCK cells can be efficiently grown from a low seeding density even in culture in a serum-free medium.

Brief Description of Drawings

FIGS. 1 are graphs for showing the results of confirmation of influenza virus growth potential in each of cell lines A and B, and Pre Cloning Cell (Example 1).
FIG. 2 is a graph for showing the results of confirmation of cell growth ability during culture of each of the cell lines A and B, and Pre Cloning Cell using a microcarrier (Example 2).
FIGS. 3 are graphs for showing the results of confirmation of medium component amounts and metabolite amounts in a culture medium during culture of each of the cell lines A and B, and Pre Cloning Cell using a microcarrier (Example 2).
FIG. 4 is a graph for showing the results of confirmation of a floating whole cell density and a cell floating ratio in a culture medium during culture of each of the cell lines A and B, and Pre Cloning Cell using a microcarrier (Example 2).
FIGS. 5 are graphs for showing the results of confirmation of an expansion factor during culture of each of the cell line A and Pre Cloning Cell using a microcarrier (Example 3).

Description of Embodiments

MDCK cells are animal cells established from the kidney of a normal male Cocker Spaniel in 1958, and have been used in various applications. The MDCK cells were revealed to have sensitivity to an influenza virus by Gaush et al. in 1968, and have been used for the growth and replication of the influenza virus as well.

A cloned MDCK cell of the present invention is a cell obtained by adapting an MDCK cell population to a serum-free medium, followed by single-cell cloning. The cloned MDCK cell of the present invention also encompasses cells obtained by growing and/or passaging cloned cells. In general, cells that have not been single-cell cloned exist as a cell population including a plurality of cells having various properties. Herein, the MDCK cell population means cells that have not been single-cell cloned. As the MDCK cell population, any MDCK cell population may be used, and an MDCK cell population obtained from a cell bank may be used. For example, cells identified as ATCC CCL-34 may be used.

The adaption of the MDCK cell population to a specific medium allows stable culture thereof in the medium. For the culture of animal cells, a medium supplemented with serum of animal origin, such as bovine or horse serum, is generally used. The serum serves as a supply source of hormones and growth factors, and is also useful in relieving a physical stress due to a culture operation. However, the use of the serum also causes many problems . One of the problems is a concern about safety. There is a risk of contamination, via the serum of animal origin, with a virus, bacteria, mycoplasma, an abnormal prion, which has caused a problem of bovine spongiform encephalopathy (BSE), or the like. Another is a concern about applicability to industrial-scale production. The serum varies in quality and composition between lots. Such lot differences affect cell growth. In addition, a large amount of the serum is needed in the industrial-scale production. However, the serum is extremely expensive, and besides, its supply is not stable. For the above-mentioned reasons, a medium containing no serum is desirably used for culturing animal cells on an industrial scale. In the present invention, in order to efficiently select a cloned MDCK cell line of MDCK cells capable of being stably cultured in a serum-free medium, a cell population has been adapted to the serum-free medium before single-cell cloning. By performing the single-cell cloning from only the cell population adapted to growth in the serum-free medium, it has become possible to selectively acquire only the cloned MDCK cell line capable of growing in the serum-free medium. The serum-free medium for the single-cell cloning may contain a growth factor or a trace element. The growth factor contained in the medium gives a cell growth signal. The kind of the growth factor in the medium in the adapting step is considered to influence characteristics of the cells. In the adapting step, cells included in the MDCK cell population are modified, while influencing each other, into cells capable of growing in the serum-free medium. In the present invention, it is considered that cells capable of efficiently growing in the serum-free medium have been able to be selected by virtue of adapting the MDCK cell population to the serum-free medium before single-cell cloning.

After the MDCK cell population adapted to the serum-free medium has been obtained, single-cell cloning is performed. In the single-cell cloning, first, the MDCK cells are isolated into single individual cells. A limiting dilution method may be used as a technique for the isolation. The isolated cloned MDCK cell line is further cultured in the serum-free medium to confluence, and passaged to provide a sufficient amount of cells. After that, the cloned MDCK cell line is evaluated for its properties, and thus a cloned MDCK cell line having suitable properties can be obtained. The cloned MDCK cell line is evaluated for, for example, its cell growth ability, seeding density, expansion factor, and sensitivity to a virus, and thus a cloned MDCK cell line having features of having a high cell growth ability, having sensitivity to a plurality of types of influenza viruses, and further having a strong adhesion force to a microcarrier can be selected. It has been difficult to select cells having all of the cell growth ability, the virus sensitivity, and the strong adhesion force to a microcarrier. However, through their extensive investigations, the inventors of the present invention have found that such cells can be selected by the above-mentioned cloning method. A technique described in Examples to be described later may be used as specific means for evaluating the cell growth ability or the like.

A culture method of the present invention has a feature in using a cell having a strong adhesion force (attachment force) to a microcarrier. The adhesion force of the cell to the microcarrier may be expressed as a cell floating ratio in the case where the cell is cultured in a culture medium containing the microcarrier. The culture method of the present invention has a feature in using a cloned MDCK cell showing a cell floating ratio of 20% or less, preferably 15% or less, more preferably 10% or less, particularly preferably 5% or less. The cell floating ratio refers to a value obtained by dividing a floating cell density obtained after culture of the MDCK cell for a certain period of time in the presence of the microcarrier by a seeding cell density, and a lower cell floating ratio indicates a higher adhesion force to the microcarrier. The cell floating ratio of the cloned MDCK cell of the present invention is a cell floating ratio shown after a lapse of 48 hours or less, preferably 24 hours or less, more preferably 6 hours or less, still more preferably 1.5 hours or less, particularly preferably 0.5 hour or less in terms of culture time. More specifically, it is preferred to use a cloned MDCK cell showing a cell floating ratio of 20% or less after a lapse of 0.5 hour, or a cell floating ratio of 5% or less after a lapse of 1.5 hours . The cell floating ratio is presumed not to significantly change depending on the cell seeding density.

The culture method of the present invention has a feature in using a cloned MDCK cell showing an expansion factor of 4.5 or more, preferably 6.5 or more, more preferably 8.5 or more. The expansion factor refers to a value obtained by dividing a cell density obtained after culture of the cell for a certain period of time by the seeding cell density, and serves as an indicator of the ease of growth . The expansion factor of the cloned MDCK cell of the present invention is an expansion factor shown during culture with the microcarrier, and is an expansion factor shown after a lapse of 48 hours or more, preferably 60 hours or more, more preferably 72 hours or more, and 144 hours or less, preferably 120 hours or less, more preferably 96 hours or less in terms of culture time. In addition, the expansion factor in the present invention is an expansion factor in the case of seeding at a seeding density of $2.0 \times 10^4$ cells/cm$^2$ or less, preferably $1.6 \times 10^4$ cells/cm$^2$ or less, more preferably $1.3 \times 10^4$ cells/cm$^2$ or less, still more preferably $1.0 \times 10^4$ cells/cm$^2$ or less, and $0.1 \times 10^4$ cells/cm$^2$ or more, preferably $0.2 \times 10^4$ cells/cm$^2$ or more, more preferably $0.3 \times 10^4$ cells/cm$^2$ or more, particularly preferably $0.6 \times 10^4$ cells/cm$^2$ or more. For example, the expansion factor may be confirmed by culturing cells using a method described in Example 3 to be described later. The culture method of the present invention has an advantage in that cells can be rapidly grown even from a low seeding density, and hence the time and cost of scale-up can be saved. In addition, according to the culture method of the present invention, the time it takes for cells to shift to a logarithmic growth phase can be saved. More specifically, it is preferred to use a cloned MDCK cell showing a cell expansion factor of 4.5 or more at from 72 hours to 96 hours of culture after seeding of the cell in the case of seeding at a seeding density of $0.6 \times 10^4$ cells/cm$^2$ or more and $2.0 \times 10^4$ cells/cm$^2$ or less.

The cloned MDCK cell in the present invention only needs to have the above-mentioned cell floating ratio and/or expansion factor, and is not particularly limited. Cells obtained by growing and/or passaging the cells selected by the above-mentioned cloning method are also encompassed in the cloned MDCK cell of the present invention. The cells may be passaged by a known technique. After cell culture with the microcarrier, the cells densely adhere onto the surface of the microcarrier as a resultof growing . In the pas sage, the densely adhering cells are transferred onto the surface of a fresh microcarrier. For example, the passage may be performed by removing the cells from the microcarrier using a protease, such as trypsin or collagenase, and then subjecting the cells to washing and the like, followed by dilution in a microcarrier-containing medium.

The cloned MDCK cell in the present invention is more specifically an MDCK cell internationally deposited and identified by accession number NITE BP-02014. Such cell was domestically deposited to NITE Patent Microorganisms Depositary (room 122, 2-5-8 Kazusakamatari, Kisarazu-shi, Chiba, Japan, postal code: 292-0818) with accession number NITE P-02014 on March 4, 2015, and then a request for conversion to an international deposit under the Budapest Treaty was made to NITE Patent Microorganisms Depositary and accepted with accession number NITE BP-02014. The MDCK cell identified by accession number NITE BP-02014 was, as described in Example 1 to be described later, selected after single-cell cloning by a limiting dilution method on the basis of MDCK cells (derived from ATCC CCL-34).

The culture method of the present invention includes culturing the cloned MDCK cell using a microcarrier. The microcarrier includes fine particles that enable cell culture by allowing cells to adhere to the surfaces thereof. The surface of the microcarrier is not particularly limited as long as a material therefor allows the cells to adhere thereto, and the microcarrier to be used in the present invention is not particularly limited. Examples of the material for the microcarrier include dextran, gelatin, collagen, polystyrene, polyethylene, polyacrylamide, glass, and cellulose. The material for the microcarrier is preferably dextran. As the shape of the microcarrier, there are given a spherical shape (beads), a disc-like shape, and the like. The shape of the microcarrier is preferably a spherical shape.

The size (diameter) of the spherical microcarrier is, for example, from about 0.01 mm to about 1 mm, preferably from about 0.05 mm to about 0.5 mm, more preferably from about 0.1 mm to about 0.3 mm. The microcarrier may be porous . Examples of the spherical microcarrier to be used in the present invention include Cytodex 1 (product name), Cytodex 3 (product name), and Cytopore (product name) (which are available from GE Healthcare Life Science). Examples of the disc-like microcarrier include Cytoline 1 (product name) and Cytoline 2 (product name) (which are available from GE Healthcare Life Science). Examples of the porous microcarrier include Cytopore (product name), Cytoline 1 (product name), and Cytoline 2 (product name) (which are available from GE Healthcare Life Science). Other commercially available examples of the microcarrier include Biosilon (product name) (NUNC), Hillex (product name) (SoloHill), and Corning (trademark) Microcarriers (Corning) . The microcarrier to be used in the present invention is particularly preferably a spherical microcarrier made of dextran. As the spherical microcarrier made of dextran, Cytodex 1 (product name), Cytodex 3 (product name), and Cytopore (product name) are preferred, Cytodex 1 (product name) and Cytodex 3 (product name) are more preferred, and Cytodex 1 (product name) is particularly preferred.

The seeding density of the cloned MDCK cell in the culture method of the present invention is not particularly limited, and may be appropriately adjusted. For example, the MDCK cells can be grown even in the case where the cells are seeded at a seeding density of $2.0 \times 10^4$ cells/cm$^2$ or less, preferably $1.6 \times 10^4$ cells/cm$^2$ or less, more preferably $1.3 \times 10^4$ cells/cm$^2$ or less, still more preferably $1.0 \times 10^4$ cells/cm$^2$ or less. In addition, the cells are desirably seeded at a seeding density of $0.1 \times 10^4$ cells/cm$^2$ or more, preferably $0.2 \times 10^4$ cells/cm$^2$ or more, more preferably $0.3 \times 10^4$ cells/cm$^2$ or more. Herein, the seeding density (cells/cm$^2$) means a cell density per culture surface area at the time of the seeding of the cells. The density of the cells in a medium may be confirmed by a known technique, and may be confirmed by, for example, a measurement method involving using a hemocytometer, an automated cell counter, or the like. In the present invention, culture is performed using the microcarrier, and hence the MDCK cells adhere to and grow on the surface of the microcarrier. Herein, the seeding density refers to a cell density per surface area of the microcarrier at the time of the seeding of the cells. When culture is performed at a seeding density as low as $2.0 \times 10^4$ cells/cm$^2$ or less, there is an advantage in that the amount of cells to be prepared for the culture can be kept to a small amount, and hence cost can be saved.

In addition, the medium for culturing the cloned MDCK cell of the present invention is not particularly limited, but is preferably a serum-free medium supplemented with no serum of animal origin. As the serum-free medium, any serum-free medium may be used, and for example, Eagle's MEM medium (Nissui Pharmaceutical Co., Ltd.), OptiPRO SFM (Thermo Fisher Scientific), VP-SFM (Thermo Fisher Scientific), EX-CELL MDCK (SAFC Biosciences), UltraMDCK (Lonza), ProVero 1 (Lonza), or BalanCD MDCK (Irvine Scientific) may be used. In addition, culture conditions, such as the density of the microcarrier, a stirring rotation speed, a dissolved oxygen concentration, and a culture temperature, only need to allow the cells to grow, and may be appropriately adjusted. In the culture method of the present invention, even when the stirring rotation speed is from about 30 rpm to about 60 rpm, the cells can satisfactorily adhere to the microcarrier and efficiently grow.

The cloned MDCK cell of the present invention may be used for, for example, a method of growing a virus capable of infecting MDCK cells, or a method of producing a substance, such as a metabolite, produced by MDCK cells. The MDCK cell of the present invention may be preferably used for a method of growing a virus capable of infecting MDCK cells. The virus capable of infecting MDCK cells only needs to be one to which MDCK cells show sensitivity, and examples thereof include an orthomyxovirus, a paramyxovirus, a rhabdovirus, and a flavivirus . A description is made by taking the case of an influenza virus as an example of the virus.

The influenza virus to which the cloned MDCK cell of the present invention shows sensitivity is not particularly limited. Examples thereof include all currently known subtypes and subtypes to be isolated and identified in the future as well. In the case of influenza A virus, which is classified into subtypes (i.e., 16 kinds of HA (H1 to H16) subtypes and 9 kinds of NA (N1 to N9) subtypes) on the basis of the antigenicity of HA molecules and NA molecules thereof, influenza viruses including combinations of the HA subtypes and the NA subtypes are conceivable. In the case of influenza B virus, influenza viruses including a combination of a Victoria lineage and a Yamagata lineage are conceivable.

Each influenza A virus subtype has high RNA genome variability, and hence new strains are frequently generated. An influenza that is said to have caused a global outbreak after being recognized as causing an outbreak in Mexico in April 2009 is called novel influenza, swine influenza, pandemic influenza A (H1N1), swine flu, A/H1N1 pdm, or the like. Novel influenza, which is said to have spread among humans after its virus, which had caused an outbreak among swine, directly infected humans from swine at farms and the like, is distinguished from Soviet influenza A (influenza A virus subtype H1N1) and Hong Kong influenza A (influenza A virus subtype H3N2), which had existed earlier and were seasonal. In addition, because of the high RNA genome variability, even in the same influenza A virus subtype, virus strains are distinguished from each other on the basis of the time and place of isolation.

Influenza B virus continues to undergo irreversible antigenic drift, but mutates relatively slower than influenza A virus, and has an outbreak cycle of about 2 years. Influenza B virus was isolated for the first time during a medium-scale influenza outbreak in New York in 1940, and has often repeated outbreaks since then, and a consequent increase in mortality rate has also been recorded. Influenza B virus has been observed to infect only humans, but has no subtypes, and has only two lineages, i.e., the Yamagata lineage and the Victoria lineage.

Other than an influenza virus isolated from a living body as described above, the influenza virus to be used in the present invention may be a recombinant virus created by adding modifications, such as attenuation, chicken egg growth adaptation, cell culture growth adaptation, modification into a temperature-sensitive phenotype, and mucosal administration adaptation, so as to be applicable to an influenza vaccine. In addition, as means for adding modifications, there are given various methods, such as: a method involving introducing mutations into eight RNA segments, such as an antigen site and a polymerase site, of an influenza virus; a method involving recombination between an RNA segment of a strain having high growth potential and an RNA segment showing antigenicity of interest by reverse genetics; a method involving generating an attenuated virus by cold-passage; and a method involving adding a mutagen to a virus culture system.

Scale-up can be performed by seeding the cloned MDCK cell in a culture medium containing the microcarrier at the above-mentioned seeding density, and then culturing the cell to confluence, followed by transfer to a fresh microcarrier. The culture time of the cloned MDCK cell in the culture method of the present invention only needs to be suited for the scale-up, and is not particularly limited. For example, the culture time is 48 hours or more, preferably 60 hours or more, more preferably 72 hours or more, and is 144 hours or less, preferably 120 hours or less, more preferably 96 hours or less. A procedure for the scale-up is similar to that for the passage.

In the culture method of the present invention, timing at which the cloned MDCK cell is infected with the influenza virus is not particularly limited, and the timing only needs to be suited for practical use. For example, it is preferred that the cell be infected with the influenza virus at the time point when the cell reaches confluence after the scale-up, and then the cell be incubated for a certain period of time. The period of time for which the cell is incubated is not particularly limited. To incubate means to maintain the cell under certain conditions for a certain period, irrespective of whether the cell grows through the incubation. The incubation may be performed under conditions similar to those under which the cell is cultured, but is preferably performed under optimal conditions for the virus with which the cell has been infected. The influenza virus with which the MDCK cell is infected is called a seed virus. The influenza virus is isolated from a living body or created by adding some modification, and is then passaged and grown using a chicken egg or any of various cells, to serve as the seed virus. The seed virus to be used in the present invention may have been passaged with any of the chicken egg and the various cells, and is not particularly limited. It is more preferred that the seed virus that has been passaged and grown in MDCK cells be allowed to infect the cloned MDCK cell in the culture method of the present invention to grow the influenza virus.

In addition, the influenza virus grown by the culture method of the present invention is used for the manufacture of an influenza vaccine. For a step of purifying the influenza virus from the MDCK cell, a known technique or any technique to be developed in the future may be used.

Examples

To help understanding of the present invention, the present invention is specifically described below by way of Examples, but the present invention is not limited to Examples.

(Example 1) Creation and Selection of Cloned MDCK Cells 1. Single-cell Cloning

(1) Culture in Serum-containing Medium

MDCK cells obtained from ATCC (ATCC No. CCL-34, Lot 1166395, number of passages: 53) were thawed, and subjected to centrifugal washing with the addition of Eagle's MEM medium (Nissui Pharmaceutical Co., Ltd.) containing 10% fetal calf serum (hereinafter referred to as FCS). The resultant pellets were caused to float by adding Eagle's MEM medium containing 10% FCS, were then cultured in a T75 flask. The cells that had been cultured at 37°C±1°C for 5 days were washed with a PBS solution containing 1 mM EDTA-4Na, and then the cells were detached from the culture vessel by adding trypsin. Eagle's MEM medium containing 10% FCS was added to neutralize trypsin, and the cells were passaged to a T225 flask. The cells were cultured at 37°C±1°C for 4 days, and then similarly passaged to a T225 flask. The cells that had been cultured at 37°C±1°C for 6 days were collected through trypsin treatment, and then Eagle's MEM medium containing 10% FCS was added to neutralize trypsin. The cells were isolated by centrifugation, and the resultant pellets were caused to float in CELLBANKER 2 (Nippon Zenyaku Kogyo Co., Ltd.) and cryopreserved in liquid nitrogen (number of passages: 56) .

(2) Adaption of ATCC-derived MDCK Cell Population to Serum-free Medium

Then, the cells obtained in (1) were thawed, and cultured in a T75 flask using serum-free medium OptiPRO SFM (Thermo Fisher Scientific). The cells that had been cultured at 37°C±1°C for 4 days were washed with a PBS solution containing 1 mM EDTA-4Na, and then detached from the culture vessel by adding TrypLE Select (Thermo Fisher Scientific) . The cells were subjected to centrifugal washing with the addition of OptiPRO SFM. The resultant pellets were caused to float by adding OptiPRO SFM, were then transferred to a T75 flask and cultured at 37°C±1°C for 4 days. The cells were collected using TrypLE Select, and subjected to centrifugal washing. The resultant pellets were caused to float in CELLBANKER 2 and cryopreserved in a freezer at -80°C (number of passages: 58).

(3) Single-cell Cloning of Serum-free AdaptedMDCK Cells

The ATCC-derived serum-free adapted MDCK cell population obtained in (2) (hereinafter referred to as Pre Cloning Cell) was subjected to single-cell cloning by a limiting dilution method, and cell lines excellent in cell growth potential and having uniform morphology were selected. A specific procedure for the cloning is as described below. Pre Cloning Cell was thawed, and cultured in a T25 flask using serum-free medium OptiPRO SFM. The cells that had been cultured at 37°C±1°C for 3 days were washed with a PBS solution containing 1 mM EDTA-4Na, and then detached from the culture vessel by adding TrypLE Select. The cells were subjected to centrifugal washing with the addition of OptiPRO SFM, and OptiPRO SFM was added to the resultant pellets to cause the cells to float again. The number of cells in the cell suspension was counted, and the cell suspension was adjusted to 5 cells/mL. The wells of a 96-well plate were each seeded with 100 μL of the 5 cells/mL cell suspension, and wells each seeded with only one cell were marked, followed by culture. After reaching confluence, the cells in the marked wells were detached with TrypLE Select and passaged to a 24-well plate. In order to remove TrypLE Select in the medium, the culture medium in the wells was completely removed and changed to a fresh medium the day after the passage. Similarly, the cells were repeatedly passaged to a 6-well plate and to a T75 flask, and the cloned MDCK cell lines were cryopreserved at the time point when a sufficient amount of cells was obtained (number of passages: 63).

2. Screening

(1) Evaluation of Growth Potential of Influenza Virus

The obtained cloned MDCK cell lines were subjected to screening using influenza virus growth potential as an indicator. In addition, in this Example, the HA titer and infectivity titer of an influenza virus were confirmed in accordance with a method disclosed in "Part IV" of "Influenza Diagnosis Manual (3rd edition, September 2014)" written by the National Institute of Infectious Diseases, Japan.

(1-1) Primary Screening

First, all the cloned MDCK cell lines and Pre Cloning Cell were thawed, and cultured in a T75 flask using serum-free medium OptiPROSFM (number of passages: 64). After thawing, the grown cloned MDCK cell lines were seeded in a 6-well plate, cultured at 37°C±1°C, and then infected with an influenza virus strain at m.o.i=0.0001 (number of passages: 65). In order to evaluate the cloned MDCK cell lines for virus growth potential, the HA titer of a virus culture supernatant was measured.

(1-2) Secondary Screening

Cloned MDCK cell lines each of which showed an HA titer equal to or higher than that of Pre Cloning Cell (30.2% of all cell lines) were evaluated for the growth potential of other influenza virus strains. In the same manner as in the previous test, cloned MDCK cell lines cultured in a 6-well plate (number of passages: 65) were infected with a virus, and then subjected to an HA titer measurement test using a virus culture supernatant, and cloned MDCK cell lines each of which showed HA titers equal to or higher than those of Pre Cloning Cell for all virus strains were selected (2.8% of all cell lines).

(1-3) Tertiary Screening

For the cloned MDCK cell lines selected by the screening using a 6-well plate, influenza virus growth potential on a T75 flask scale was further confirmed. Cloned MDCK cell lines cultured in a T75 flask (number of passages: 65) were each infected with each of virus strains, such as A/New Caledonia (H1N1) and A/Hiroshima (H3N2), at m.o.i=0.0001. The HA titer of a virus culture supernatant was confirmed, and cloned MDCK cell lines each of which showed HA titers equal to or higher than those of Pre Cloning Cell for all virus strains were selected (0.6% of all cell lines).

(1-4) Quaternary Screening

For the cloned MDCK cell lines selected by the screening using a T75 flask, the influenza virus growth potential of cells passaged for 10 or more generations was confirmed. The cloned MDCK cell lines were thawed, and subcultured in a T75 flask using serum-free medium OptiPRO SFM. The cells that had reached a number of passages of from 77 to 80 were infected with each of virus strains, such as A/New Caledonia (H1N1), at m.o.i=0.0001. The infectivity titer of a virus culture supernatant was measured, and the results revealed that each of the cloned MDCK cell lines attained infectivity titers of 7.0 $\log_{10}TCID_{50}$/mL or more for all virus strains.

(2) Evaluation of Cell Growth Ability on Microcarrier

The cloned MDCK cell lines selected using virus growth potential as an indicator were evaluated for suitability to microcarrier culture.

(3) Selection of Cloned MDCK Cell Line

On the basis of the results of the above-mentioned evaluation, an ATCC-derived serum-free adapted cloned MDCK cell line A (hereinafter referred to as cell line A) capable of being cultured in a serum-free medium, and having sensitivity to influenza viruses of a plurality of subtypes, and further having a strong adhesion force to a microcarrier was selected. Cytodex 1 (GE Healthcare Life Science) was used as the microcarrier. Meanwhile, a cloned MDCK cell line B (hereinafter referred to as cell line B) having a weaker adhesion force to the microcarrier than the cell line A, but similar to the cell line A in the other properties was also selected and used for comparison.

The cell line A was deposited with accession number NITE P-02014 to NITE Patent Microorganisms Depositary (room 122, 2-5-8 Kazusakamatari, Kisarazu-shi, Chiba, Japan, postal code: 292-0818) (date of acceptance: March 4, 2015), and a request for conversion to an international deposit under the Budapest Treaty was made to NITE Patent Microorganisms Depositary and accepted with accession number NITE BP-02014.

(4) Influenza Virus Growth Potential of Selected Cloned MDCK Cell Line

The cell line A was grown in a T75 flask. At the time point when the cell line A reached confluence, the cells were inoculated with an influenza virus. Parameters regarding culture conditions for cells and parameters regarding the number of cells at the time of infection with an influenza virus strain are as shown below.

Table 1

| Kin d of cells | Number of passages | Seeding density (cells/mL) | Culture vessel | Culture conditions Culture period | Number of cells reached (cells/ flask) |
|---|---|---|---|---|---|
| Pre Cloning Cell | P64 | $5 \times 10^4$ | 75 cm$^2$ vented flask (30mL culture) | 37°C, 5% $CO_2$, 3 days | $1,230 \times 10^4$ |
| Cell line B | P73 | $6 \times 10^4$ | | | $1,135.5 \times 10^4$ |
| Cell line A | P74 | $4.5 \times 10^4$ | | | $1,980 \times 10^4$ |

All virus strains used for inoculation were provided by the National Institute of Infectious Diseases, Japan and passaged for 5 generations in MDCK cells, and are called A/Ibaraki/N12073/2011(H1N1)pdm09 (hereinafter referred to as TA-73), A/Ibaraki/N12232/2012(H3N2) (hereinafter referred to as TA-232), B/Ibaraki/N12322/2012 (hereinafter referred to as TA-322), and B/Ibaraki/N12336/2012 (hereinafter referred to as TA-336), respectively. The inoculation amount is m.o.i=0.001. Eagle's MEM medium supplemented with 4 mM glutamine, 4.7 g/L glucose, 20 mM sodium hydrogen carbonate, and 0.1×TrypLE Select was used as a culture medium, and culture was performed under the conditions of 34°C and 5% $CO_2$. After virus infection, a culture supernatant on each of day 1 to day 3 of culture was sampled, and measured for an HA titer and an infectivity titer. The HA titer was measured through a reaction with 0.5% chicken erythrocytes at room temperature for 1 hour for each of TA-73, TA-322, and TA-336, and through a reaction with 1.0% guinea pig erythrocytes at 4°C for 1.5 hours for TA-232. In addition, the unit of the infectivity titer is $\log_{10}TCID_{50}$/mL.

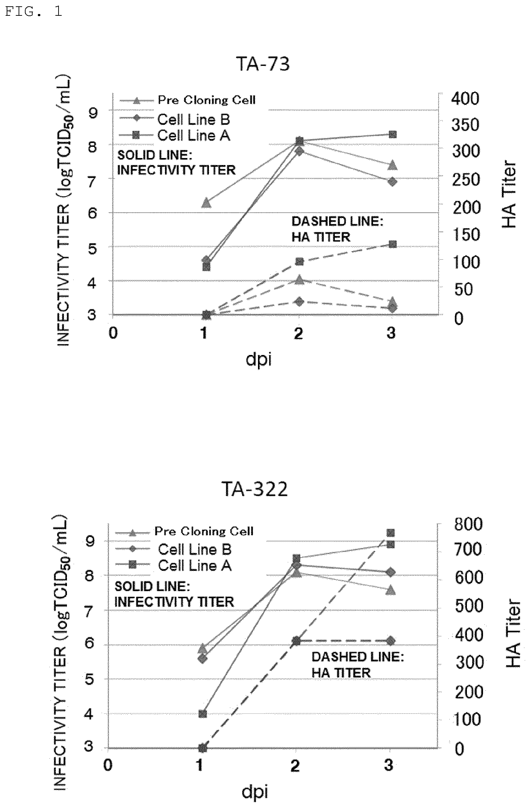

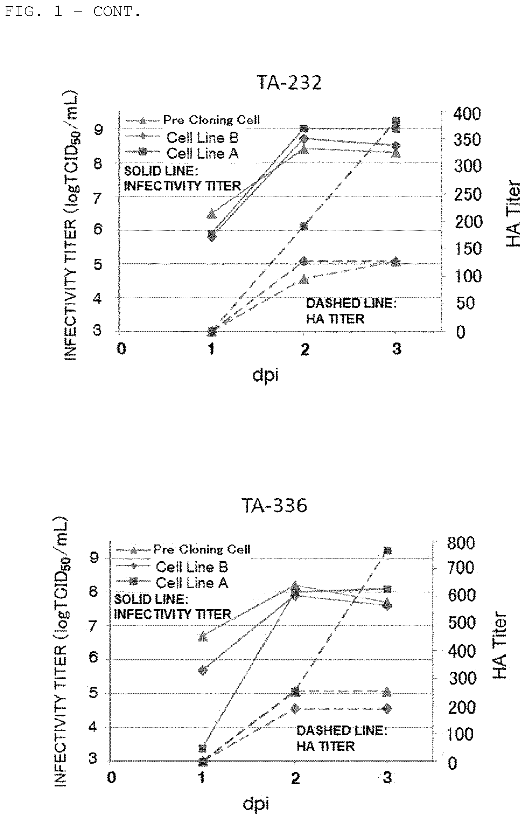

The results of the HA titer on day 1 to day 3 (1 dpi to 3 dpi) after influenza virus infection are shown in Table 2 below, and the results of the infectivity titer thereon are shown in Table 3 below. In addition, those results are summarized as graphs shown in FIGS. 1. In FIGS. 1, a solid line represents the infectivity titer, and a dashed line represents the HA titer.

Table 2

| HA titer | | | | | |
|---|---|---|---|---|---|
| cell | virus | 1 dpi | 2 dpi | 3 dpi |
| Pre Cloning Cell | TA-73 (A/H1N1pdm09) | <2 | 64 | 24 |
| | TA-232 (A/H3N2) | <2 | 96 | 128 |
| | TA-322 (B/Vic) | <2 | 384 | 384 |
| | TA-336 (B/Yam) | <2 | 256 | 256 |
| Cell line B | TA-73 (A/H1N1pdm09) | <2 | 24 | 12 |
| | TA-232 (A/H3N2) | <2 | 128 | 128 |
| | TA-322 (B/Vic) | <2 | 384 | 384 |
| | TA-336 (B/Yam) | <2 | 192 | 192 |
| Cell line A | TA-73 (A/H1N1pdm09) | <2 | 96 | 128 |
| | TA-232 (A/H3N2) | <2 | 192 | 384 |
| | TA-322 (B/Vic) | <2 | 384 | 768 |
| | TA-336 (B/Yam) | <2 | 256 | 768 |

Table 3

| Infectivity titer | | | | | |
|---|---|---|---|---|---|
| cell | virus | 1 dpi | 2 dpi | 3 dpi |
| Pre Cloning Cell | TA-73 (A/H1N1pdm09) | 6.3 | 8.1 | 7.4 |
| | TA-232 (A/H3N2) | 6.5 | 8.4 | 8.3 |
| | TA-322 (B/Vic) | 5.9 | 8.1 | 7.6 |
| | TA-336 (B/Yam) | 6.7 | 8.2 | 7.7 |
| Cell line B | TA-73 (A/H1N1pdm09) | 4.6 | 7.8 | 6.9 |
| | TA-232 (A/H3N2) | 5.8 | 8.7 | 8.5 |
| | TA-322 (B/Vic) | 5.6 | 8.3 | 8.1 |
| | TA-336 (B/Yam) | 5.7 | 7.9 | 7.6 |

(continued)

| Infectivity titer | | | | |
|---|---|---|---|---|
| cell | virus | 1 dpi | 2 dpi | 3 dpi |
| Cell line A | TA-73 (A/H1N1pdm09) | 4.4 | 8.1 | 8.3 |
| | TA-232 (A/H3N2) | 5.9 | 9.0 | 9.0 |
| | TA-322 (B/Vic) | 4.0 | 8.5 | 8.9 |
| | TA-336 (B/Yam) | 3.4 | 8.0 | 8.1 |

On the basis of the transitions of the HA titer and the infectivity titer, each of the virus strains was confirmed to have a tendency to show higher influenza virus growth potential when grown in the cell line A than when grown in any other cell line.

(Example 2) Culture of Cloned MDCK Cell Line using Microcarrier

The cell line A was cultured in serum-free medium OptiPRO SFM (Thermo Fisher Scientific) supplemented with 4 mM glutamine. The cell line B and Pre Cloning Cell were used as control cells. The cells were seeded at a seeding density of $2.3 \times 10^4$ cells/cm². Cytodex 1 (GE Healthcare Life Science) was used as a microcarrier at a density of 3.5 g/L. Culture conditions were as follows: stirring rotation speed: 15 rpm until 48 hours of culture and 30 rpm from 48 hours onward, pH: 7.0, temperature: 37.0°C, and dissolved oxygen concentration (DO) : 3.00 ppm. Aeration was performed with a porous tube. In addition, a bioreactor having a volume of 3 L was used as a culture vessel.

The number of cells in the culture medium was confirmed by a technique involving sampling part of the culture medium, and dispersing cells adhering to the microcarrier using trypsin . A cell viability autoanalyzer (Beckman Coulter) was used for the measurement of the number of cells. In addition, the concentration of a metabolite was confirmed using a bioprocess analyzer Cedex Bio (Roche Diagnostics).

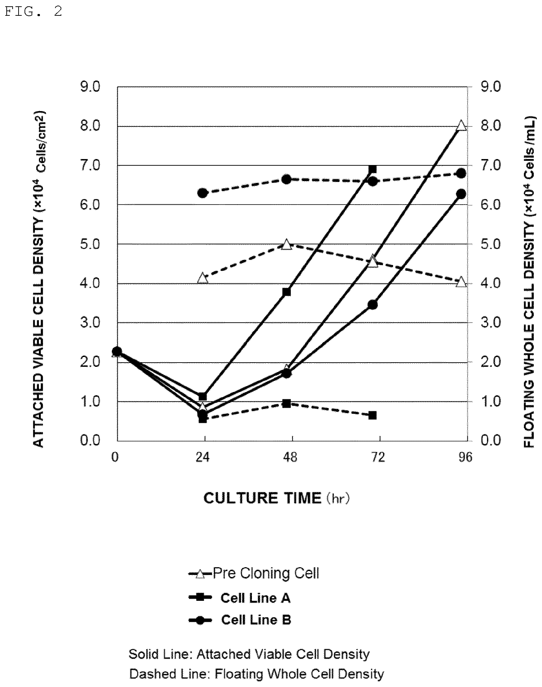

The results of confirmation of the cell growth ability of each cell line are shown in FIG. 2. In FIG. 2, a solid line represents the number of cells adhering to the microcarrier, and a dashed line represents the number of cells floating in a culture supernatant. Each cell line cannot grow and dies in a floating state . The cell line A had a smaller number of cells floating in the supernatant as compared to Pre Cloning Cell or the cell line B, and was thus confirmed to have a stronger adhesion force to the microcarrier.

The cell line A grew to $6.9 \times 10^4$ cells/cm² on day 3 of culture. Meanwhile, the cell line B and Pre Cloning Cell each took 4 days to grow to the same degree.

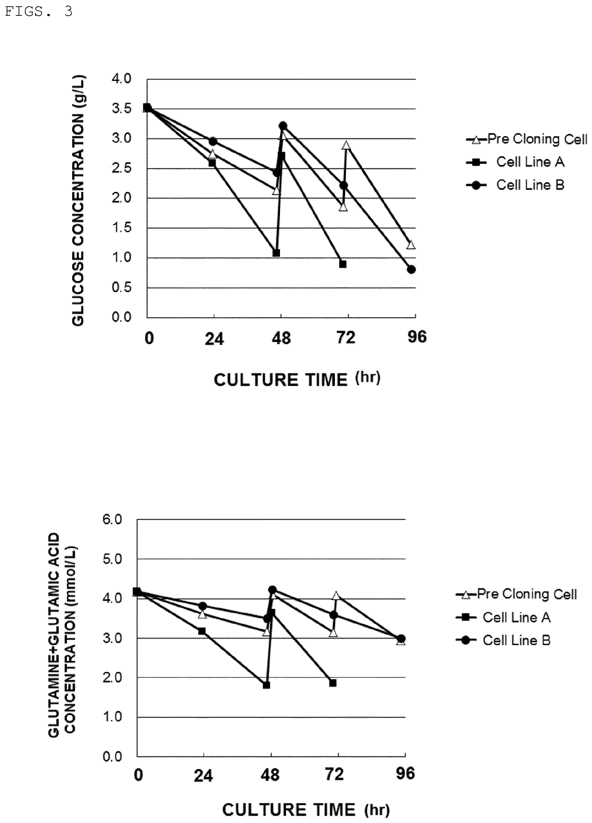

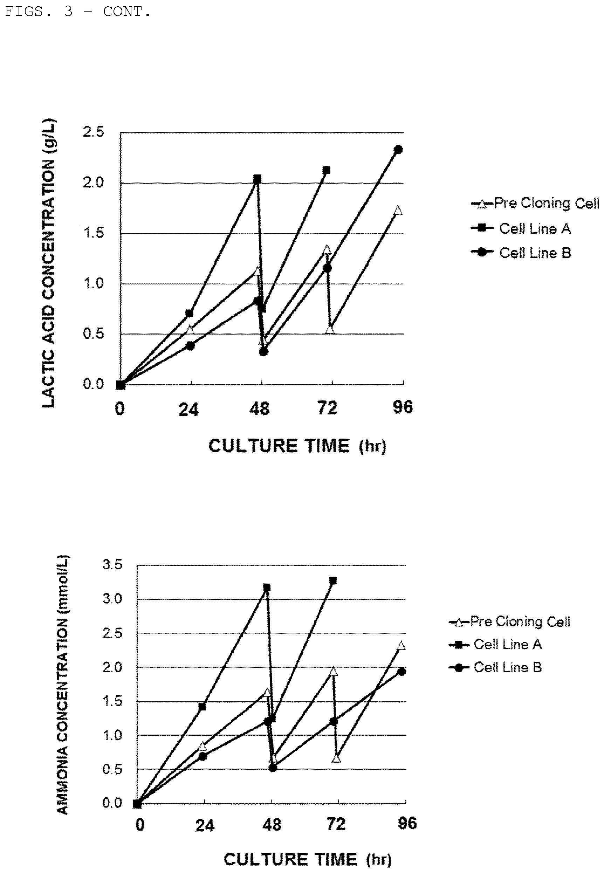

The concentrations of medium components and cell metabolites present in the medium are shown in FIGS. 3.

In the culture medium of the culture conditions of this Example, the number of cells floating in the supernatant was measured. The ratio of cells floating in the supernatant to the seeded cells was calculated by the following equation, and defined as a cell floating ratio.

```
(Floating whole cell density at each culture time)÷(seeding cell

density)×100=cell floating ratio (%)
```

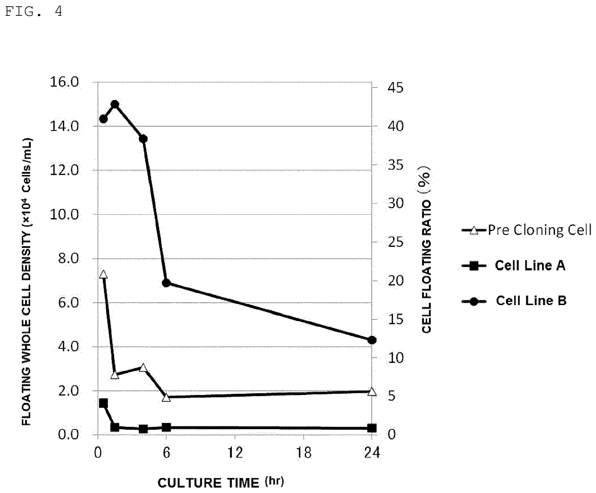

The results of confirmation of the floating whole cell density of each cell line are shown in FIG. 4, and the cell floating ratio of each cell line is shown in Table 4 below. The cell line A had a lower cell floating ratio as compared to Pre Cloning Cell or the cell line B, and was thus confirmed to have a stronger adhesion force to the microcarrier. In addition, it is considered that, in the cell line A, almost all the seeded cells are attached to the microcarrier after a lapse of 1.5 hours from the start of culture.

Table 4

| Cell floating ratio | | | | | |
|---|---|---|---|---|---|
| Culture time (hr) | 0.5 | 1.5 | 4 | 6 | 24 |
| Pre Cloning Cell | 20.9 | 7.8 | 8.8 | 4.9 | 5.6 |
| Cell line A | 4.1 | 1.0 | 0.8 | 1.0 | 0.9 |

(continued)

| Cell floating ratio | | | | | |
|---|---|---|---|---|---|
| Cell line B | 41.0 | 42.9 | 38.4 | 19.7 | 12.3 |

(Example 3) Confirmation of Expansion Factor of Cloned MDCK Cell Line

The cell line A was seeded at the following cell seeding densities a to d and cultured in the same manner as in Example 2. Pre Cloning Cell was used as a control.

a: $2.0 \times 10^4$ cells/cm$^2$ ($17.6 \times 10^4$ cells/mL)
b: $1.0 \times 10^4$ cells/cm$^2$ ($8.8 \times 10^4$ cells/mL)
c: $0.6 \times 10^4$ cells/cm$^2$ ($5.3 \times 10^4$ cells/mL)
d: $0.3 \times 10^4$ cells/cm$^2$ ($2.6 \times 10^4$ cells/mL)

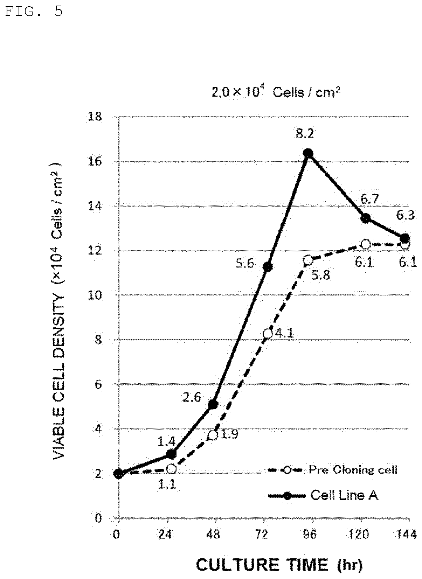

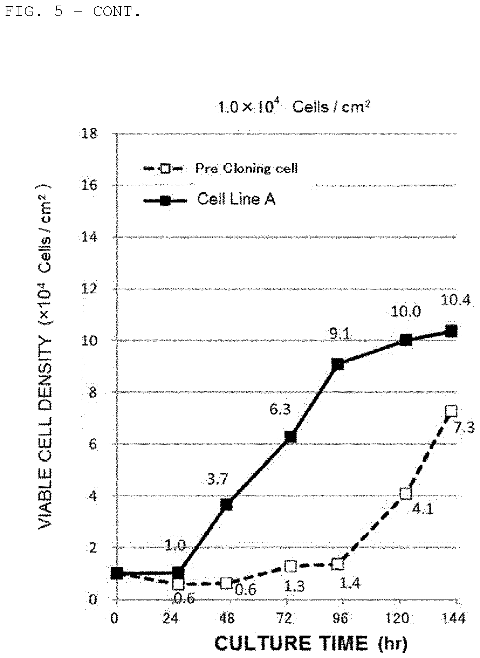

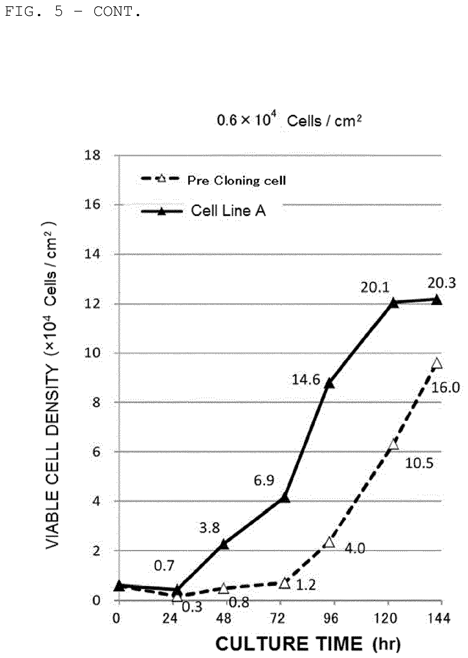

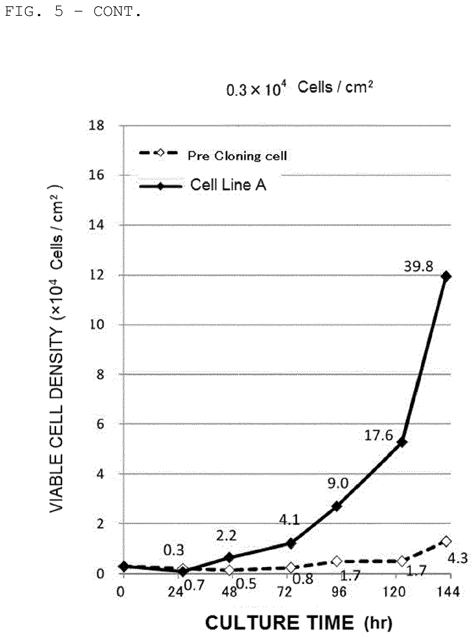

The culture volume was 400 mL, and stirred culture was performed at 60 rpm at 37.0°C in the presence of 5% $CO_2$. Cytodex 1 was used as a microcarrier. The density of the microcarrier is 2.0 g/L.
The results are shown in FIGS. 5. A value near each point of the graphs represents the value of an expansion factor at the corresponding time point. The cell line A had higher expansion factors after a lapse of from about 30 hours to about 144 hours from the start of culture as compared to Pre Cloning Cell, and was thus found to be capable of more efficiently growing. In addition, the cell line A showed expansion factors of about 4.5 or more after a lapse of from about 72 hours to about 96 hours from the start of culture irrespective of the cell seeding density, and was found to show higher values as compared to Pre Cloning Cell.

(Example 4) Transfer of Cloned MDCK Cell Line between Microcarriers

The cell line A was transferred from microcarrier to microcarrier under the following conditions. In addition, after the transfer, the cells were infected with an influenza virus, and the HA titer and infectivity titer of the virus were measured by the same method as in Example 1.

(1) Method for Transfer between Microcarriers

The cell line A was grown by being cultured in a serum-free medium on a 2 L scale using Cytodex 1 as a microcarrier. After that, controls of culture conditions were stopped and the microcarrier was allowed to settle. After that, the culture supernatant was removed, and was washed at 37°C for 30 minutes with the addition of a PBS solution containing 1 mM EDTA-4Na. The microcarrier was allowed to settle again, and then the cell washing solution was removed. Trypsin was added, and treatment was performed at 37°C for about 30 minutes. A trypsin inhibitor was added, and then the whole amounts of the cells and the microcarrier were transferred to a 20 L culture vessel. Seeding parameters at the time of cell seeding are shown in Table 5, and growth parameters during cell culture are shown in Table 6.

Table 5

| Seeding parameters | | | | |
|---|---|---|---|---|
| | First test | | Second test | |
| Working volume | 2 L | 20 L | 2 L | 20 L |
| Concentration of microcarrier (MC) | 4.5 g/L | 3.5 g/L | 5.0 g/L | 3.5 g/L |
| Amount of microcarrier | 9.0 g | 70.0 g | 10.0 g | 70.0 g |
| Seeding density (cells/cm$^2$) | $1.31 \times 10^4$ | $2.19 \times 10^4$ | $1.82 \times 10^4$ | $1.71 \times 10^4$ |

Table 6

| Growth parameters | | |
| --- | --- | --- |
| Working volume | 2 L | 20 L |
| Stirring | 20 rpm to 30 rpm | 20 rpm to 60 rpm |
| Temperature | 37.0°C | 37.0°C |
| pH | 7.4 or less | 7.4 or less |

(2) Virus Sensitivity of Cloned MDCK Cell Line after Transfer between Microcarriers

After the transfer between microcarriers, the MDCK cells were inoculated with an influenza virus at the time point when the cells reached confluence. The virus strain used for inoculation is TA-73, and the inoculation amount is m.o.i=0.001. At the time of the influenza virus inoculation, the working volume was 20 L, and the viable cell density was from $12.8 \times 10^4$ cells/cm$^2$ to $16.0 \times 10^4$ cells/cm$^2$. Eagle's MEM medium supplemented with 4 mM glutamine, 3.6 g/L glucose, 20 mM sodium hydrogen carbonate, and $0.1 \times$ TrypLE Select was used as a medium. After the virus inoculation, culture was performed under the culture conditions of a stirring rotation speed of from 20 rpm to 60 rpm, a pH of 7.0, a temperature of 34.0°C, and a dissolved oxygen concentration (DO) of 3.00 ppm. After virus infection, the culture supernatant was sampled daily until day 4 of culture, and measured for an infectivity titer. Of those, the highest value of the infectivity titer was defined as a peak infectivity titer ($\log_{10}$TCID$_{50}$/mL). The measurement of the infectivity titer was performed in the same manner as in Example 1.
The peak infectivity titer was as shown in Table 7 below.

Table 7

| Peak infectivity titer | | |
| --- | --- | --- |
| Virus strain | First test | Second test |
| TA-73 | 8.50 | 8.72 |

The cell line A showed a high cell growth ability in microcarrier culture, and was thus found to enable the preparation of a large amount of cells at the start of virus culture, and to show high virus growth potential even though the microcarrier culture repeated.

Industrial Applicability

As described in detail above, according to the culture method of the present invention, MDCK cells can be efficiently grown using a microcarrier. Further, through the use of the culture method of the present invention, a virus can be efficiently grown, and hence a vaccine can be efficiently manufactured. Thus, the culture method of the present invention is industrially excellent. Large-volume culture is essential in achieving practical use in vaccine manufacture. In this regard, according to the MDCK cell and the method of culturing the cell with a microcarrier of the present invention, there is an advantage in that the cost and time of scale-up can be saved by virtue of a high expansion factor on the microcarrier and/or a high adhesion force to the microcarrier.

**Claims**

1. A method of culturing a cloned MDCK cell showing an expansion factor of 4.5 or more when cultured using a microcarrier.

2. A method of culturing a cloned MDCK cell according to claim 1, wherein the cloned MDCK cell shows a cell floating ratio of 20% or less.

3. A method of culturing a cloned MDCK cell according to claim 1, wherein the expansion factor of the cloned MDCK cell comprises an expansion factor in a case where the cloned MDCK cell is seeded at a cell seeding density of $2.0 \times 10^4$ cells/cm$^2$ or less and cultured.

**4.** A method of culturing a cloned MDCK cell according to claim 1 or 3, wherein the expansion factor of the cloned MDCK cell comprises an expansion factor in a case where the cloned MDCK cell is cultured for 48 hours or more.

**5.** A method of culturing a cloned MDCK cell according to any one of claims 1 to 4, wherein the cloned MDCK cell comprises a cell obtained by adapting an MDCK cell population to a serum-free medium, followed by cloning.

**6.** A method of culturing a cloned MDCK cell, comprising culturing an MDCK cell identified by accession number NITE BP-02014 using a microcarrier.

**7.** A method of growing a virus, comprising using the method of culturing a cloned MDCK cell of any one of claims 1 to 5.

**8.** A method of growing a virus according to claim 7, further comprising infecting an MDCK cell with a virus, and incubating the MDCK cell.

**9.** A method of growing a virus according to claim 7 or 8, wherein the virus comprises an influenza virus.

**10.** A cloned MDCK cell showing an expansion factor of 4.5 or more when cultured using a microcarrier.

**11.** A cloned MDCK cell according to claim 10, wherein the expansion factor of the cloned MDCK cell comprises an expansion factor in a case where the cloned MDCK cell is seeded at a cell seeding density of $2.0 \times 10^4$ cells/cm$^2$ or less and cultured.

**12.** A cloned MDCK cell according to claim 10 or 11, wherein the expansion factor of the cloned MDCK cell comprises an expansion factor in a case where the cloned MDCK cell is cultured for 48 hours or more.

**13.** A cloned MDCK cell according to any one of claims 10 to 12, wherein the cloned MDCK cell comprises a cell obtained by adapting an MDCK cell population to a serum-free medium, followed by cloning.

FIG. 1

TA-73

TA-322

FIG. 1 - CONT.

TA-232

TA-336

FIG. 2

Solid Line: Attached Viable Cell Density
Dashed Line: Floating Whole Cell Density

FIGS. 3

FIGS. 3 - CONT.

FIG. 4

FIG. 5

$2.0 \times 10^4$ Cells / cm²

FIG. 5 – CONT.

FIG. 5 - CONT.

$0.6 \times 10^4$ Cells / cm²

FIG. 5 – CONT.

$0.3 \times 10^4$ Cells / cm²

## INTERNATIONAL SEARCH REPORT

| International application No. |
|---|
| PCT/JP2017/012726 |

### A. CLASSIFICATION OF SUBJECT MATTER
*C12N5/071*(2010.01)i, *C12N7/00*(2006.01)i

According to International Patent Classification (IPC) or to both national classification and IPC

### B. FIELDS SEARCHED

Minimum documentation searched (classification system followed by classification symbols)
C12N5/071, C12N7/00

Documentation searched other than minimum documentation to the extent that such documents are included in the fields searched
Jitsuyo Shinan Koho          1922–1996   Jitsuyo Shinan Toroku Koho   1996–2017
Kokai Jitsuyo Shinan Koho    1971–2017   Toroku Jitsuyo Shinan Koho   1994–2017

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS/WPIX(STN)

### C. DOCUMENTS CONSIDERED TO BE RELEVANT

| Category* | Citation of document, with indication, where appropriate, of the relevant passages | Relevant to claim No. |
|---|---|---|
| X/Y | GEORGE Meena, et al., "Production of Cell Culture (MDCK) Derived Live Attenuated Influenza Vaccine (LAIV) in a Fully Disposable Platform Process", Biotechnol. Bioeng., 2010, Vol.106, No.6, pp.906-917, see "Cell Line" on p.907, Table II, Fig. 3 | 1-5,7-13/ 1-5,7-13 |
| X/Y | GENZEL, Y., et al., "Wave microcarrier cultivation of MDCK cells for influenza virus production in serum containing and serum-free media", Vaccine, 2006, Vol.24, pp.6074-6087, see "Cell line" on p.6075, "Cell numbers/pH value" on p.6078 | 1-5,7-13/ 1-5,7-13 |

☒ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 June 2017 (13.06.17) | 27 June 2017 (27.06.17) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

**INTERNATIONAL SEARCH REPORT**

International application No.

PCT/JP2017/012726

C (Continuation). DOCUMENTS CONSIDERED TO BE RELEVANT

| Category* | Citation of document, with indication, where appropriate, of the relevant passages | Relevant to claim No. |
|---|---|---|
| X/Y | GENZEL Y. et al., "Serum-free influenza virus production avoiding washing steps and medium exchange in large-scale microcarrier culture", Vaccine, 2006, Vol.24, pp.3261-3272, see "Cell line" on p.3262, "Online data/cell numbers" on p.3266, Fig. 2(E) | 1-5,7-13/ 1-5,7-13 |
| X/Y | GENZEL Yvonne, et al., "MDCK and Vero cells for influenza virus vaccine production: a one-to-one comparison up to lab-scale bioreactor cultivation", Appl. Microbiol. Biotechnol., 2010, Vol.88, pp.461-475, see "Cell lines" on p.462, Fig. 5b | 1-5,7-13/ 1-5,7-13 |
| X/Y | SUGO Ken, et al., "HYDROXYAPATITE MICROCARRIER (2)", Tiss. Cult. Commun., 2006, Vol.25, pp.113-118, see Fig. 3 | 1-5,7-13/ 1-5,7-13 |
| X/Y | HU Alan Yung-Chih, et al., "Production of Inactivated Influenza H5N1 Vaccines from MDCK Cells in Serum-Free Medium", PLOS ONE, 2011, Vol.6, No.1, e14578, see Fig. 2 | 1-5,7-13/ 1-5,7-13 |
| X/Y | MERTEN O.-W., et al., "Production of Influenza Virus in Serum-Free Mammalian Cell Cultures", Dev. Biol. Stand., 1999, Vol.98, pp.23-37, see Fig. 3 | 1-5,7-13/ 1-5,7-13 |
| X/Y | Yan-yu ZHANG et al., "Optimizing the serum-free micro- carrier culture condition of MDCK cells and influenza Virus H1N1", Journal of Yunnan University, 2011, vol.33, no.3, pages 340 to 344, see Fig. 2 | 1-5,7-13/ 1-5,7-13 |
| X/Y | Qing-shuai FAN et al., "Preliminary Optimization of Production Condition of Rabies Virus with MDCK Cells Cultured in Serum-free Medium", Chin. J. Biologicals, 2009, vol.22, no.11, pages 1136 to 1140, see Fig. 2 | 1-5,7-13/ 1-5,7-13 |
| X/Y | JP 2012-503486 A (MedImmune, L.L.C.), 09 February 2012 (09.02.2012), claim 1; fig. 1 & US 2010/0098725 A1 & WO 2010/036760 A1 & EP 2334328 A1 & KR 10-2011-0074563 A & CN 102215865 A | 1-5,7-13/ 1-5,7-13 |
| X/Y | US 2010/0136647 A1 (GE HEALTHCARE BIO-SCIENCES), 03 June 2010 (03.06.2010), fig. 2 (Family: none) | 1-5,7-13/ 1-5,7-13 |

Form PCT/ISA/210 (continuation of second sheet) (January 2015)

**INTERNATIONAL SEARCH REPORT**

International application No.

PCT/JP2017/012726

C (Continuation).    DOCUMENTS CONSIDERED TO BE RELEVANT

| Category* | Citation of document, with indication, where appropriate, of the relevant passages | Relevant to claim No. |
|---|---|---|
| Y | WO 2006/095431 A1  (Kyoritsu Seiyaku Corp.), 14 September 2006 (14.09.2006), paragraphs [0020] to [0036]; examples 1 to 2 & US 2008/0138362 A1    & EP 1862537 A1 & CA 2601006 A | 1-5,7-13 |
| Y | JP 2010-503412 A  (MedImmune, L.L.C.), 04 February 2010 (04.02.2010), claims 8, 10 & US 2008/0286850 A1    & WO 2008/105931 A2 & EP 2069480 A2        & KR 10-2009-0057444 A & CN 101627113 A | 1-5,7-13 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016065251 A
- WO 1997037000 A1
- WO 2008105931 A2
- WO 2010036760 A1

**Non-patent literature cited in the description**

- **NATIONAL INSTITUTE OF INFECTIOUS DISEAS-ES.** Influenza Diagnosis Manual. September 2014